# EUROPEAN PATENT SPECIFICATION

(11) **EP 0 606 804 B1**
(45) Date of publication and mention of the grant of the patent: **04.10.2001**
(21) Application number: 93403185.7
(22) Date of filing: 27.12.1993
(51) Int. Cl.: G01N 33/533, G01N 33/48, C07K 7/08, G01N 15/14, G01N 33/68, G01N 33/58

(54) **Marker for neurotensin receptor**
Marker für Neurotensinrezeptor
Marqueur pour le récepteur de neurotensine

(30) Priority: 30.12.1992 CA 2086453
(43) Date of publication of application: 20.07.1994
(73) Proprietor: MARTINEX R & D INC., Montreal, Québec H3A 1B1 (CA)
(72) Inventor: Beaudet, Alain, Quebec H3P 1B2 (CA); Faure, Marie-Pierre, Saint-Laurent H4R 1M2 (CA); Gaudreau, Pierrette, Brossard, Quebec J4X 1V5 (CA)
(74) Representative: Peaucelle, Chantal

(56) References cited:
- EP-A- 0 333 071
- THE JOURNAL OF BIOLOGICAL CHEMISTRY, vol.248, no.19, 10 October 1973, BALTIMORE, MD pages 6854 - 6861 CARRAWAY ET AL. 'The isolation of a new hypotensive peptide, neurotensin, ....'
- THE JOURNAL OF NEUROSCIENCE, vol.7, no.10, October 1987, NEW YORK, NY pages 3088 - 3104 SCHAFFNER ET AL. 'Fluorescence-activated cell sorting of embryoic mouse ....'
- Biochem. Biophys. Res. Comm., vol. 120, 1984, pp.:206-213
- Developmental Brain Research, vol. 61, 1991, pp: 259-264

### SEQUENCE LISTING

(1) GENERAL INFORMATION:
   (i) APPLICANT: McGill University
      845 Sherbrooke Street West
      Montreal, Quebec, Canada H3A 1B1
   (ii) TITLE OF INVENTION: MARKER FOR NEUROTENSIN RECEPTOR
   (iii) NUMBER OF SEQUENCES: 3
   (iv) CORRESPONDENCE ADDRESS:
      (A) ADDRESSEE: Cabinet Armengaud Aine
      (B) STREET: 3, avenue Bugeaud
      (C) CITY: Paris
      (E) COUNTRY: France
      (F) ZIP: 75116
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: 93403185.7
      (B) FILING DATE: 27-DEC-1993
      (C) CLASSIFICATION:
   (vii) PRIOR APPLICATION DATA:
      (A) APPLICATION NUMBER: CA 2,086,453
      (B) FILING DATE: 30-DEC-1992
   (viii) ATTORNEY/AGENT INFORMATION:
      (A) NAME: PEAUCELLE, Chantal
      (C) REFERENCE/DOCKET NUMBER: 57682-290
   (ix) TELECOMMUNICATION INFORMATION:
      (A) TELEPHONE: (1) 45.53.05.50
      (B) TELEFAX: (1) 45.53.80.21
      (C) TELEX: 642586 ARMEN
(2) INFORMATION FOR SEQ ID NO:1:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (ix) FEATURE:
      (A) NAME/KEY: Modified-site
      (B) LOCATION: 4
      (D) OTHER INFORMATION: /note= "The exact identity of position 4 is not yet determined, it is either Tyr or Trp"
   (xi) SEQUENCE DESCRIPTION: SEQ ID NO:1:
   Arg Arg Pro Xaa Ile Leu
   1               5
(2) INFORMATION FOR SEQ ID NO:2:
   (i) SEQUENCE CHARACTERISTICS:
      (A) LENGTH: 6 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (ix) FEATURE:
      (A) NAME/KEY: Modified-site
      (B) LOCATION: 1
      (D) OTHER INFORMATION: /note= "Position 1 is Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg or
   (ix) FEATURE:
      (A) NAME/KEY: Modified-site
      (B) LOCATION: 4
      (D) OTHER INFORMATION: /note= "The exact identity of position 4 is not yet determined, it is either Tyr or Trp"
   (xi) SEQUENCE DESCRIPTION: SEQ ID NO:2:
   Xaa Arg Pro Xaa Ile Leu
   1               5
(2) INFORMATION FOR SEQ ID NO:3:
   (i) SEQUENCE CHARACTERISTICS:
      (A) LENGTH: 13 amino acids
      (B) TYPE: amino acid
      (C) STRANDEDNESS: single
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO:3:
   Glu Leu Tyr Glu Asn Lys Pro Arg Arg Pro Tyr Ile Leu
   1               5                   10

## Description

### BACKGROUND OF THE INVENTION

### a) Field of the Invention

The invention relates to a selective fluorescent peptidic probe for neurotensin receptors, a method for *in vitro* labeling of neurotensin receptors on cell surface and a method to isolate neurotensin-receptor expressing cells.

### b) Description of Prior Art

Neurotensin (NT) is a peptide of 13 amino acids (pGlu-Leu-Tyr-Glu-Asn-Lys-Pro-Arg-Arg-Pro-Tyr-Ile-Leu-OH⁾ isolated from the hypothalamus (Carraway, R. & Leeman, S.E., The J. of Biol. Chem., 1973, 248:6854-6861) and subsequently identified in the central nervous system (CNS) and the gastrointestinal tract of all vertebrates. Neurotensin fulfills a dual function as a neuromodulator in the nervous system and as a hormone in the gastrointestinal tract. Both modes of action imply as a first step the selective association of the neuropeptide with a specific receptor located on the plasma membrane of target cells.

Neurotensin-binding sites have been localized and characterized in various tissue preparations and in cell cultures from central and peripheral nervous systems. Pharmacological studies have demonstrated the existence of two populations of neurotensin binding sites : a low affinity and high capacity binding component (NTR1), from which neurotensin binding is displaced by the potent histamine antagonist, levocabastine; and a high affinity and low capacity binding components (NTR2) corresponding to the physiologically relevant form of the receptor. Both rat and human NTR2 components have been cloned and sequenced (Tanaka, Masu, and Nakanishi, Neuron, 1990, 4:947-854), Some of the biochemical events mediating neurotensin signal transduction have also been characterized.

In recent years, neurotensin derivatives that may be useful as central nervous system depressants and be administered orally or by injection, have been described (Atsuhiko et al., EP-A-033011). In addition, fluoro-labeled probes have been developed for localizing peptide, cytokine, drug, and hormone receptors at the cellular level in tissue and cell cultures (Hazum E., Change K-J, & Cuatrecas P., Proc. Natl. Acad. Sciences USA, 1979, 77:3038-3041). This concept, which involves labeling purified molecules covalently with fluorochromes such as fluorescein, has permitted the characterization of the kinetics, the distribution and the ultimate fate of many ligands in living cells (Taylor D.L. & Wang Yu-Li, Nature, 1980, 284:405-410).

It would be highly desirable to be provided with a non-toxic highly sensitive tool for biochemical, pharmacological and anatomical studies of the neurotensin receptor in both brain and peripheral tissues.

All markers for neurotensin receptors derived to date are of radioactive nature and hence have a limited half-life. In addition, radioactive probes for neurotensin receptors are costly and provide only static information on underlying biological [proceses]processes.

Further, it would be highly desirable to be provided with a marker for neurotensin receptor which would allow for the isolation of neurotensin-receptor expressing cells. This application is of particular interest in view of the demonstration of a selective association of neurotensin receptors with midbrain dopaminergic cells and with basal forebrain cholinergic cells ( Annals of the New York, Academy of Sciences, "Neurobiology of Neurotensin", volume 668, Editors : P. Kitabgi and Charles Nemeroff. New York Academy of Sciences, New York, New York (1992). Thus the isolation of neurotensin-receptor expressing cells would make it possible to sort out and selectively administer dopaminergic or cholinergic cells in diseases such as Parkinson's and Alzheimer's disease , respectively. The treatment of these diseases would be greatly improved if the population of cells administered were homogenous, since dissociated embryonic central nervous system preparations may contain cells that may otherwise interfere with the treatment.

Embryonic mouse and rat motoneurons have been isolated by Schaffner et al. using flow cytometry (The J. of Neuroscience, 1987, 7:3088-3104). These isolated cells were found to be 80% pure with respect to the presence of fluorescent label in cells examined with fluorescent microscopy immediately after sorting. These results suggest that isolated neurons from human embryonic tissue may be administered to patients in need of brain grafts.

It would be also highly desirable to be provided with a marker for neurotensin receptor which allows for receptor physiological studies in tissue slices and in cell cultures and for distinguishing cell surface from intracellular receptor components.

### SUMMARY OF THE INVENTION

One aim of the present invention is to provide for a non-toxic highly sensitive and selective marker of the neurotensin receptor for biochemical, pharmacological and anatomical studies of the said receptor in both brain and peripheral tissues.

Another aim of the present invention is to provide for a marker of neurotensin receptors which would allow for the isolation of neurotensin-receptor expressing cells.

Another aim of the present invention is to provide for a marker of neurotensin receptors which allows for receptor physiological studies *in vivo* or *in vitro* and for distinguishing cell surface from intracellulor receptor components.

In accordance with the present invention there is provided a compound having the formula: 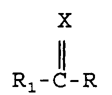 or a pharmaceutical acceptable acid salt thereof, wherein
R is a polypeptide moiety comprising an amino acid sequence: wherein Y is selected from the group consisting of Arg, Pro-Arg, A-Pro-Arg, Asn-A-Pro-Arg, GLu-Asn-A-Pro-Arg, B-Glu-Asn-A-Pro-Arg,Leu-B-Glu-Asn-A-Pro-Arg or D-Leu-B-Glu-Asn-A-Pro-Arg wherein
   A is Lys or Orn;
   B is Tyr or tyr;
   D is Glu or pGlu; (SEQ ID No.:2) and
   Z is Tyr or Trp;
R₁ is a light-emitting fluorophore;
   and
X is oxygen or sulfur,
   wherein R₁ is linked to R via an N α-amino group or an amino-terminal amino acid residue of Y and wherein said amino acid residue is selected from the group consisting of D, Leu, B, Glu, Asn, Pro, Lys, or Orn.

In accordance with the present invention there is provided for *in vitro* labeling of neurotensin receptors on cell surface, which comprises the steps of:
a) incubating dissociated cells, cell culture preparations or tissue slices with a compound of the present invention; and
b) visualizing said compound of step a) using flow cytometry and, or epifluorescence/confocal microscopic methods.

In accordance with the present invention there is also provided a method to isolate neurotensi-receptor expressing cells, which comprises the steps of:
a) incubating dissociated cells or cell culture preparations with a compound of the present invention;
b) isolating cells bound to the compound of the present invention using flow cytometric cell-sorting methods.

### BRIEF DESCRIPTION OF THE DRAWINGS

Fig. 1 is the structur of N-Fluoresceyl thiocarbamyl-[Glu¹] neurotensin, in accordance with one embodiment of the present invention;
Fig. 2 is an analytical chromatographic profile of a compound of the present invention and of neurotensin;
Fig. 3 is a confocal optical section of a cholinergic.hybrid (SN6) cell incubated with a compound of the present invention;
Fig. 4 is a displacement curve of the binding of a compound of the present invention on mouse brain membranes by iodinated-neurotensin;
Fig. 5 is a light microscopic image of fluoro-neurotensin labeling of dopaminergic neurons in rat brain sections; and
Fig. 6 is a light microscopic image of fluoro-neurotensin labeling of dopaminergic neurons in rat brain sections.

### DETAILED DESCRIPTION OF THE INVENTION

In the present invention the amino acids are identified by the conventional three-letter abbreviations as indicated below:

| | |
|---|---|
| Alanine | Ala |
| Arginine | Arg |
| Asparagine | Asn |
| Aspartic Acid | Asp |
| Cysteine | Cys |
| Glutamic Acid | Glu |
| Glycine | Gly |
| Histidine | His |
| Leucine | Leu |
| Lysine | Lys |
| Methionine | Met |
| Ornithine | Orn |
| Phenylalanine | Phe |
| Proline | Pro |
| Serine | Ser |
| Threonine | Thr |
| Tryptophane | Trp |
| Tyrosine | Tyr |
| D-Tyrosine | tyr |
| Valine | Val |

The present invention relates to highly sensitive fluorescent probes which allow for rapid and precise characterization of neurotensin receptor binding properties on whole cells.

The fluorescent compounds of the present invention have the following general formula:

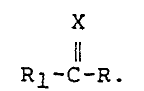

R is a polypeptide moiety which consists essentially of an amino acid sequence selected from the group consisting of:

In accordance with the present invention, each of the amino acid residues identified at positions 1 to 8 may be substituted by Lys or Orn.

Further, the amino acid sequence of the polypeptide moiety in accordance with the present invention may be lengthened at the N- or C-terminal as long as the neurotensin-like biological activity is preserved.

In accordance with the present invention, the expression neurotensin-like biological activity is intended to mean that the polypeptide induces biological effects similar to those of neurotensin and/or binds with high affinity and selectivity to the neurotensin receptor.

R₁ is a fluorophore moiety selected from the group consisting of fluorescein, such as fluorescein isothiocyanate, 5-carboxy-fluorescein, 6-carboxy-fluorescein, rhodamine , such as tetramethyl rhodamine isiothiocyanate, Blue fluorescent, such as BODIPY™, and Texas red.

In accordance with the present invention, other fluorophores may be used where neurotensin-like biological activity is preserved.

In accordance with the present invention, the fluorophores may be linked to the polypeptide moiety at position ranging from 1 to 8 via a thiocarbamyl bond, where X is sulfur, or a peptide bond, where X is oxygen.

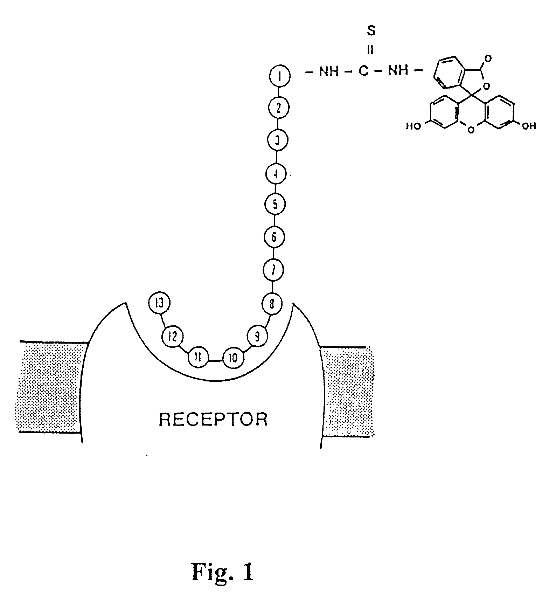

The preferred compound in accordance with the present invention is N-fluoresceyl thiocarbamyl-[Glu¹] neurotensin (N-FTC-[Glu¹]NT) as shown in Fig. 1.

Although there have been previous attempts at conjugating peptides with fluorescein, N-FTC-[Glu¹]NT is the first example of a successful conjugation of fluorescein with the tridecapeptide neurotensin. The salient features of one compound of the present invention are:
(1) the selective attachment of the fluorescein molecule to the neurotensin N-terminus;
(2) the purification of the conjugated compound to approximately 99% purity allowing for optimal detection sensitivity;
(3) the similarity of its pharmacological properties with those of the native peptide; and
(4) the fact that it is 100% non-toxic and has a demonstrated shelf life of at least one year.

The fluorescent peptide compounds of the present invention offer a new, inexpensive and highly sensitive tool for biochemical, pharmacological and anatomical studies of the neurotensin receptor in both brain and peripheral tissues. The present fluorescent probes offer several advantages over the use of radioactive compounds.

The compounds of the present invention do not have any of the common drawbacks of radioactive molecules such as short half-life, high cost and slow detection yield (which may imply weeks of photographic exposure). Further, they compensate for two major shortcomings of current neurotensin radioactive probes: their low specific activity (which admittedly is higher with iodinated than tritiated ligands, but also entails greater biohazards) and the fact that they essentially provide static information (i.e. information that is not applicable to studying living processes in real time).

In addition to providing a non-radioactive approach to the characterization of neurotensin receptors, the fluorescent compounds of the present invention may be used for a number of additional applications unsuited to radioactive probes. These include the following:
(1) these fluorescent compounds may be readily applied to the isolation of neurotensin-receptor expressing cells, using flow cytometric cell-sorting methods. Similarly, receptor binding studies may be carried out on whole cells by flow cytometry.
(2) The fluorescent compounds of the present invention may be used for real time visualization of physiological processes (receptor aggregation, capping and internalization) using confocal laser microscopy on brain slices or in cell culture preparation. The same technique may be used for distinguishing cell surface with respect to intracellular components.
(3) Confocal microscopic visualization of the bound fluorescent compounds may be combined with that of other cell markers (e.g. Biocytin™, Lucifer™ yellow) for identification of neurotensin receptors on electro-physiologically recorded cells. It may also be conjugated to the immunocytochemical characterization of the cells and/or compartments harboring the labeled receptors, using appropriate fluorescent-tagged antibodies.

In accordance with one embodiment of the present invention, N-FTC-[Glu¹]NT is prepared according to the following procedure.

### 1- Fluorescent labeling of neurotensin

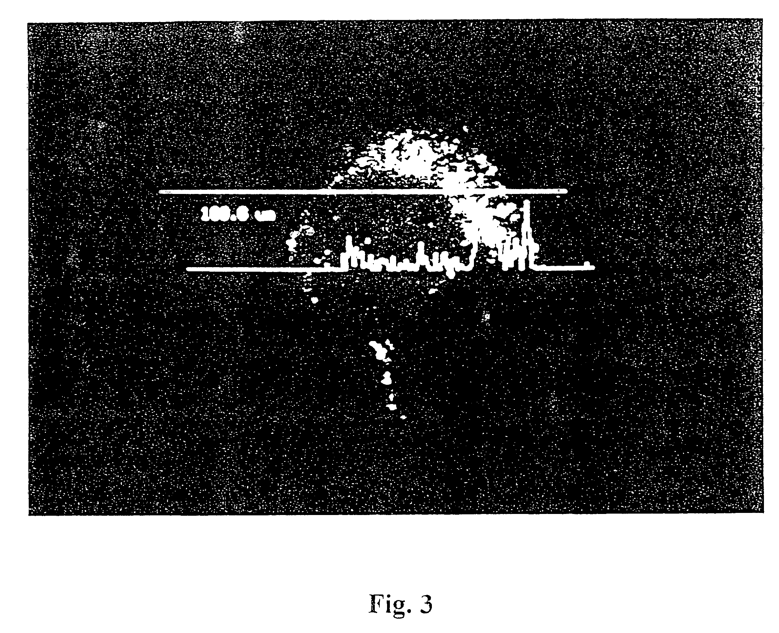

[Glu¹]NT was synthesized by solid phase technique using a scheme based on t-Boc chemistry/acid labile amino acid protecting groups. After deprotection of the last N-amino group, acylation was performed by fluorescein isothiocyanate (FITC, Sigma, 6-fold excess) in anhydrous dimethylformamide (DMF) containing 5% N,N-diisopropylethylamine (DIEA) for 2 hours at room temperature with stirring. Completion of the coupling was ascertained by a ninhydrin colorimetric test. The acyl-peptide-resin intermediate was then extensively washed with DMF and dried *in vacuo*. It was submitted to hydrogen fluoride cleavage to deprotect amino acid side chains and to cleave the fluorescein thiocarbamyl (FTC) peptide from the resin. The FTC-peptide was solubilized in trifluoroacetic acid (TFA) and subjected to rotary evaporation *in vacuo*. It was then purified by preparative high pressure liquid chromatography (HPLC) on a parsil 10 ODS-3 Whatman™ column (10-um particle size; 2.2 cm x 50 cm), using a binary solvent system consisting of 0.01% aqueous TFA, pH 2,9 and acetonitrile (CH₃CN)-0.01% TFA and an appropriate gradient. Elution of the peptide was monitored at 214nm. Collected fractions were readily screened by analytical HPLC using both UV and fluorescence detection (excitation, 338 nm; emission, 425 nm), pooled accordingly, evaporated *in vacuo* to remove CH₃CN and lyophilized twice. The purified N-FTC-[Glu1]NT was analyzed for homogeneity by analytical HPLC on a u Bondapak™ C₁₈ column (10-um particle; 0.39 cm x 15 cm) using appropriate linear gradients of 0.01% aqueous TFA; pH 2.9 and 0.01% TFA-CH₃CN and 0.01M ammonium acetate and CH₃CN (Fig. 3, where the position of [Glu¹]NT and its fluorescent analog FTC-[Glul]NT are indicated on the profiles). Its amino acid composition was assessed by quantitative amino acid analysis after acidic hydrolysis *in vacuo* (6N HCl, 110°C, 18h) and carboxypeptidase Y (CPY) digestion (6U/0.3umole, 37°C, 48h).

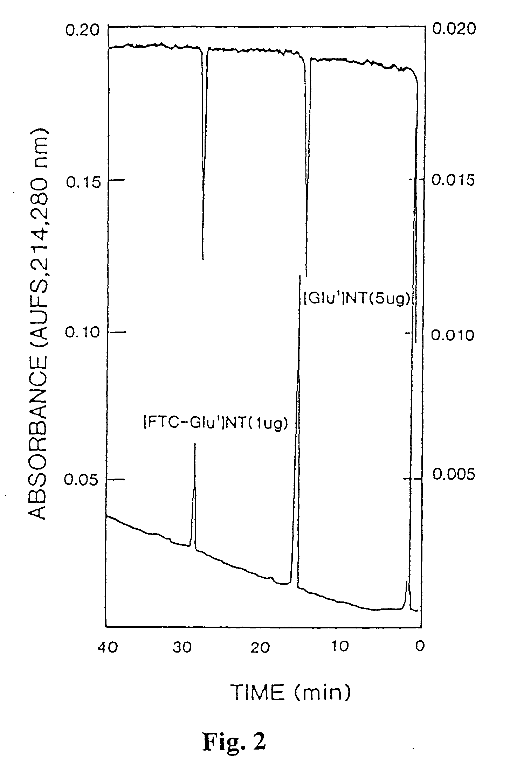

The site of attachment of the fluoresceyl derivative molecule to the neurotensin N-terminus was identified as Nα-Glu¹. The structure of the fluorescent peptide was confirmed by mass spectral analysis. The degree of homogeneity was determined by U.V. and fluorescence detection to 99%. The modification of semi-protected neurotensin with FITC yielded a selective incorporation of one mole FITC/mole unprotected peptide. N-FTC-[Glu¹]NT was evaluated to be pure as indicated by a single elution peak from reverse-phase HPLC allowing for optimal detection sensitivity (Fig.2). The molecular weight of N-FTC-[Glu¹]NT is 2080. The coumpound is freely soluble in distilled water or aqueous buffer, and is stable if protected from light and maintained at 4°C. Finally, FTC-NT is 100% non-toxic.

### 2-Competitive binding assay

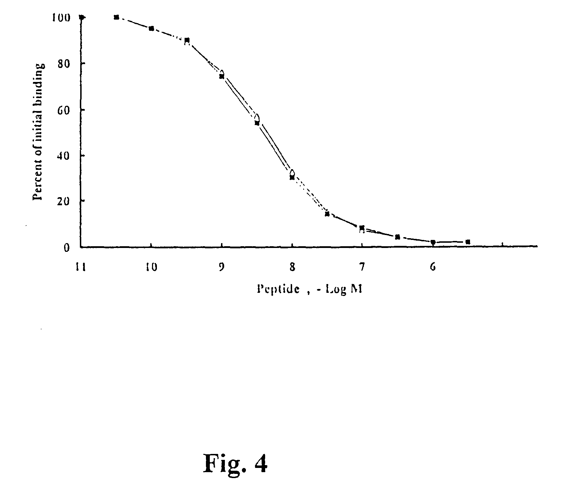

The binding of monoiodo ¹²⁵I-Tyr₃-neurotensin was performed on purified brain membranes from adult male mice as described previously (Sadoul J.L. et al., Biochem. and Biophys. Res. Comm., 1984, 120(1):206-213). Briefly, membranes were incubated with 0.1 nM of radiolabeled peptide in the presence of varying concentrations of N-FTC-[Glu¹]NT in 50 mM Tris HCl pH 7.5 containing 0.2% bovine serum albumin and 1 mM 1,10-phenanthroline. The reaction was carried out for 20 minutes at 22°C and stopped by the addition of 2 ml ice-cold buffer. Membranes were then subjected to immediate filtration over Gelman™-filters (Millipore™) under vacuum using a Millipore™ filtration apparatus. They were then thoroughly washed and their radioactivity content was measured in a gamma counter. The data were expressed as the percentage of specific binding of the radioligand in the absence of competitor. IC₅₀ values were obtained graphically and then corrected for the occupancy by the labeled ligand to obtain Kᵢ values. The Kᵢ values presented are the geometric mean [anti-log of averaged log (Kᵢ) values] +/- SEM. The data were analyzed on an IBM/XT microcomputer using EBDA/LIGANG programs. As can be seen in Figure 4, the fluorescent analogue completely displaces specific 125_{I}-Tyr₃-neurotensin binding in a dose dependent manner. Scatchard analysis of the data indicates that the binding is virtually the same as that of native neurotensin with an IC₅₀ of 0.55nM and a pseudo-Hill coefficient of approximately 1.

### Example I

### In vitro labeling of neurotensin receptors on Rat brain tissue sections

Rats were sacrificed by decapitation, the brains were rapidly removed, blocked in the coronal plane and frozen at -40°C. 25µm-thick frozen sections of the substantia nigra were cut on a cryostat and incubated with 10⁻⁴-10⁻⁶ M fluoro-neurotensin diluted in binding buffer (pH 7.4). The incubations were performed at 4°C in the dark for 60 minutes to allow for equilibration, after which the sections were rapidly rinsed in phosphate-buffered-saline (4 x 60 seconds each), dipped in double-distilled water, and air dried under a cool stream of air. The distribution of the fluorescent labeling was examined under a Leica Diaplan™ microscope using a high pressure 100-W mercury lamp and the appropriate dichroic filter combinations for excitation/emission of fluorescein (485/520 nm).

Controls for these experiments included: (1) examination of sections incubated in the absence of the fluorescent ligand to determine background autofluorescence, and in the presence of a 1000-fold excess of unlabeled ligand for the determination of the non-specific binding; and (2) examination of regions of the nervous system known to be devoid of neurotensin receptors.

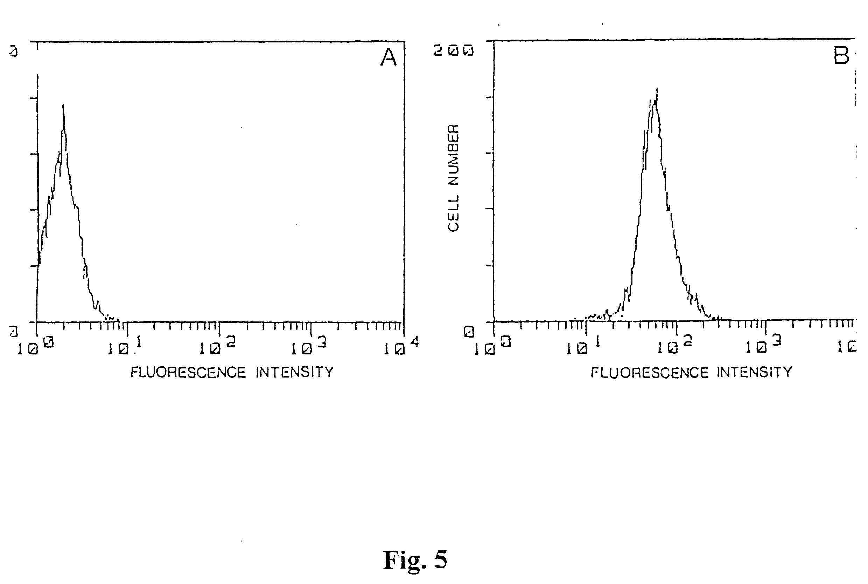

The anatomical distribution of specifically bound fluorescent ligand in the ventral midbrain tegmentum is illustrated in Fig. 5.

In the substantia nigra pars compacta the label is seen to be selectively accumulated over nerve cell bodies and proximal dendrites. The labeled neurons are ovoid and fusiform in shape with their long axis oriented parallel to the dorsal surface of the pars reticulata. In the latter, the label is mainly confined to a few scattered perikaria, however several labeled processes are seen to radiate from the cells in the pars compacta.

In the ventral tegmental area, the labeling is intense and associated with both nerve cell bodies and surrounding neuropil. In the parabrachial pigmentous division, the labeling is interrupted by areas devoid of label corresponding to the trans-tegmental fiber bundles. The fluorescent labeling was no longer apparent in sections incubated with an excess of unlabeled neurotensin. No autofluorescence was observed except a few orange spots in some cells, typical of lipofuscin aggregates.

### Example II

### Flow cytometric analysis of fluoro-neurotensin binding to cholinergic hybrids cells

Hybridomas cells (SN6) were produced by the fusion of embryonic septal cells with murine neuroblastoma and generously provided by Hammond et al. (Science, December 1986, 234:1237-1240). SN cells were grown in petri dishes (100 mm²) in Dubelcco's modified Eagle's medium (DMEM) containing 44 mM NaHCO₃ and 10% fetal calf serum (Gibco BRL) in a humidified atmosphere of 90% air, 10% CO₂ at 37°C.

For flow cytometry, the cells (1x10⁶cell/0.1 ml) were washed in PBS and incubated with various concentrations of fluoro-neurotensin in Hepes-Tris-buffer, pH 7.4, in the presence (non-specific) or the absence (total binding) of a 100-fold excess of unlabeled neurotensin. After incubation at 4°C or 20°C for 1 hour, the cells were washed and analyzed with a Becton-Dickinson™ Facscan flow cytometer and consort 30 software.

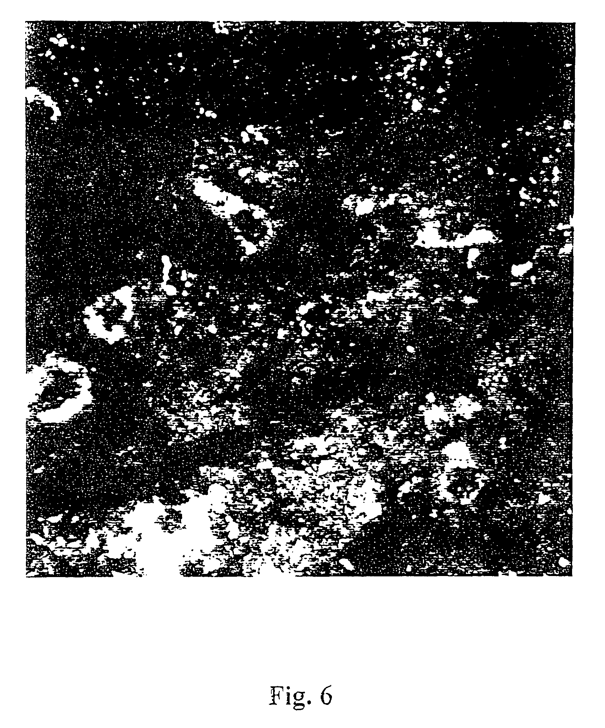

Flow cytometric histograms of N-FTC-[Glu¹]NT binding to SN6 cholinergic hybrid cells are illustrated in Fig. 6. The majority (97.4%) of the cells displayed specific fluoro-neurotensin binding at both 4°C and 20°C. Saturation of the binding was both time and temperature dependent. Maximal binding densities were higher at 4°C than at 20°C, presumably reflecting a down regulation of cell surface receptors subsequent to internalization.

### Example III

### Confocal microscopic visualization of neurotensin binding sites on cholinergic hybrid cells.

SN6 cholinergic hybrid cells were incubated with N-FTC-[Glu¹]NT, under the same conditions as above and the distribution of bound fluorescent molecules was analyzed by confocal microscopy.

Confocal imaging was performed with a Leica™ confocal laser scanning microscope configured with a Leica Diaplan™ inverted microscope (equipped with a 40 x NPL oil immersion fluotar objective of 1.30 numerical aperture), an argon ion laser (488 nm) with an output power of 2-50mW, and a VME bus with an MC 68020/68881™ computer system integrated to an optical disc for image storage. All image generating and processing operations were performed on a Leica™ confocal laser microscope software. Optical scanning images (512 x 512 pixels) of cultured cells were made at 0.1 µm intervals for a total of 26 sections per scanning sequence. From this data volume, a single composite image of each cell was generated using extended focus image construction.

Confocal laser microscopic examination of cells incubated with fluoro-neurotensin at 4°C showed prominent staining of the cell borders, suggesting confinement of the label to the cell membrane. Upon warming of these cells up to 37°C for 10 minutes, the surface fluorescence intensity diminished and multiple small, bright fluorescent particles appeared in the cytoplasm. After 30 min at 37°C, these fluorescent endosome-like elements accumulated and formed a perinuclear ring as illustrated in Fig. 2.

While the invention has been described with particular reference to the illustrated embodiment, it will be understood that numerous modifications thereto will appear to those skilled in the art. Accordingly, the above description and accompanying drawings should be taken as illustrative of the invention and not in a limiting sense.

## Claims

1. A compound having a formula: 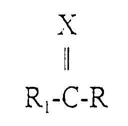 or a pharmaceutically acceptable acid salt thereof, wherein R is a polypeptide moiety comprising an amino acid sequence:
wherein Y is selected from the group consisting of Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg or D-Leu-B-Glu-Asn-A-Pro-Arg wherein
A is Lys or Orn;
B is Tyr or tyr;
D is Glu or pGlu; (SEQ ID No.: 2) and
Z is Tyr or Trp;
R₁ is a light-emitting fluorophore;
and
X is oxygen or sulfur;
wherein R₁ is linked to R via an N α-amino group or an amino-terminal amino acid residue of Y and wherein said amino acid residue is selected from the group consisting of D, Leu, B, Glu, Asn, Pro, Lys, or Orn.

2. A compound according to claim 1, wherein Y is Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg or C-Leu-B-Glu-Asn-A-Pro-Arg wherein,
A is Lys or Orn;
B is Tyr or try; and
C is Glu or pGlu, (SEQ ID No: 2);
and said R₁ is linked to said R via an amino acid residue of Y and that each of said amino acid residue selected from the group consisting of C, Leu, B, Glu, Asn₂ and Pro may be substituted by Lys or Orn.

3. A compound according to claim 1, wherein said polypeptide is neurotensin or an analog thereof.

4. A compound according to claim 1, wherein R is Glu-Leu-Tyr-Glu-Asn-Lys-Pro-Arg-Arg-Pro-Tyr-Ile-Leu, (SEQ ID N° 3), 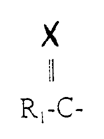 is fluorescein thiocarbamyl and R is linked via the N-terminus of position 1.

5. A compound having the formula : 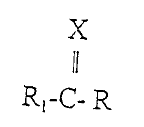 or a pharmaceutically acceptable acid salt thereof, wherein R is an amino acid sequence comprising at least amino acid residues 8-13 of neurotensin, R₁ is a light-emitting fluorophore ; and X is oxygen or sulfur.

6. A compound according to anyone of claims 1 to 5, wherein said light-emitting fluorophore is selected from the group consisting of fluorescein, rhodamine, Blue fluorescent, and Texas red.

7. A compound according to anyone of claims 1 to 6, wherein said compound is at least 99 % homogenous.

8. A method of *in vitro* labelling of neurotensin receptors on a cell surface, said method comprising incubating dissociated cells, cell culture preparations or tissue slices with a compound according to anyone of claims 1 to 7, and visualizing said compounds using flow cytometric and/or epifluorescence/confocal microscopic methods.

9. A method according to claim 8, wherein said visualizing further comprises observing said compound and other fluorescent markers for histochemical electrophysiological, or histochemical and electrophysiological identification of neurotensin receptor-containing cells.

10. A method of isolating neurotensin receptor cells, said method comprising : incubating dissociated cells or cell culture preparations with a compound according to claim 1 or claim 5, and isolating cells bound to said compound using flow cytometric cell-sorting methods.

## Patentansprüche

1. Eine Verbindung mit der Formel: 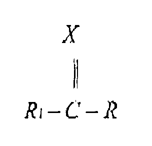 oder ein pharmazeutisch brauchbares Säuresalz derselben, worin R eine Polypeptideinheit ist, welche eine folgende Aminosäuresequenz umfaßt:
worin Y ausgewählt ist aus der Gruppe Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg oder D-Leu-B-Glu-Asn-A-Pro-Arg, worin
A Lys oder Orn;
B Tyr oder tyr;
D Glu oder pGlu, (SEQ ID No. : 2) und
Z Tyr oder Trp sind;
R₁ ein lichtemitierender Fluorophor,
und
X Sauerstoff oder Schwefel sind;
worin R₁ an R über N einer α-Aminogruppe oder einen aminoterminalen Aminosäurerest von Y gebunden ist und worin der Aminosäurerest ausgewählt ist aus der Gruppe D, Leu, B, Glu, Asn, Pro, Lys oder Orn.

2. Verbindung nach Anspruch 1, worin Y Arg, Pro-Arg, A-Pro-Arg, Asn-A-Pro-Arg, Glu-Asn-A-Pro-Arg, β-Glu-Asn-A-Pro-Arg, Leu-B-Glu-Asn-A-Pro-Arg oder C-Leu-B-Glu-Asn-A-Pro-Arg ist, worin
A Lys or Orn;
B Tyr oder tyr; und
C Glu oder pGlu, (SEQ ID No. : 2) ist;
und R₁ an R über einen Aminosäurerest von Y gebunden ist und jeder der Aminosäurereste, die ausgewählt sind aus der Gruppe C, Leu, B, Glu, Asn und Pro, substituiert sein kann durch Lys oder Orn.

3. Verbindung nach Anspruch 1, worin das Polypeptid Neurotensin oder ein Analoges desselben ist.

4. Verbindung nach Anspruch 1, worin R Glu-Leu-Tyr-Glu-Asn-Lys-Pro-Arg-Arg-Pro-Tyr-Ile-Leu (SEQ ID No.: 3) ist, 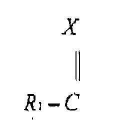 Fluorescein-Thiocarbamyl ist und R über das endständige N der Position 1 gebunden ist.

5. Verbindung mit der Formel: 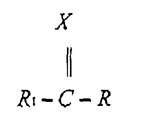 oder ein pharmazeutisch brauchbares Säuresalz derselben, worin R eine Aminosäuresequenz ist, die wenigstens Aminosäurereste 8-13 von Neurotensin umfaßt, R₁ ein lichtemittierender Fluorophor, und X Sauerstoff oder Schwefel sind.

6. Verbindung nach einem der Ansprüche 1 bis 5, worin der lichtemittierende Fluorophor ausgewählt ist aus der Gruppe Fluorescein, Rhodamin, Fluoreszenzblau und Texasrot.

7. Verbindung nach einem der Ansprüche 1 bis 6, worin die Verbindung wenigstens 99 % homogen ist.

8. Verfahren zum Tn-Vitro-Markieren von Neurotensinrezeptoren auf einer Zelloberfläche, wobei bei diesem Verfahren vereinzelte Zellen, Zellkulturpräparationen oder Gewebeschnitte mit einer Verbindung nach einem der Ansprüche 1 bis 7 inkubiert werden und die Verbindungen unter Verwendung von Durchflußzytometrie und/oder Epifluoreszenz/konfokalen mikroskopischen Methoden visuell erkannt werden.

9. Verfahren nach Anspruch 8, worin das visuelle Erkennen außerdem die Beobachtung der Verbindung und anderer fluoreszierender Markierungen für histochemische elektrophysiologische oder histochemische und elektrophysiologische Identifizierung von Neurotensin-Rezeptor-enthaltenden Zellen umfaßt.

10. Verfahren zum Isolieren von Neurotensinrezeptorzellen, wobei dissoziierte Zellen oder Zellkulturpreparationen mit einer Verbindung nach Anspruch 1 oder Anspruch 5 inkubiert werden und an die Verbindung gebundene Zellen unter Verwendung von durchflußzytometrischen Zellsortiermethoden isoliert werden.

## Revendications

1. Composé de formule : 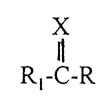 ou un sel d'acide pharmaceutiquement acceptable de celui-ci, dans lequel R est un fragment de polypeptide qui comprend la séquence d'acides aminés :
dans laquelle Y est choisi dans le groupe constitué par Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg ou D-Leu-B-Glu-Asn-A-Pro-Arg, où
A représente Lys ou Orn ;
B représente Tyr ou tyr ;
D représente Glu ou pGlu ; (SEQ ID No.: 2) et
Z représente Tyr ouTrp ;
R₁ est un fluorophore photoémetteur ;
et
X représente un atome d'oxygène ou de soufre ;
où R₁ est lié à R par l'intermédiaire d'un groupe N α-amino ou d'un résidu d'acide aminé à terminaison amino, de Y et où ledit résidu d'acide aminé est choisi dans le groupe constitué par D, Leu, B, Glu, Asn, Pro, Lys ou Orn.

2. Composé selon la revendication 1, dans lequel Y représente Arg, Pro-Arg, A-Pro-Arg, Asn-A-Pro-Arg, Glu-Asn-A-Pro-Arg, B-Glu-Asn-A-Pro-Arg, Leu-B-Glu-Asn-A-Pro-Arg, ou dans lequel
A représente Lys ou Orn ;
B représente Tyr ou try ; et
C représente Glu ou pGlu ; (SEQ ID No: 2) ;
et ledit R₁ est lié audit R par l'intermédiaire d'un résidu d'acide aminé de Y et chacun desdits résidus d'acide aminé choisi dans le groupe constitué par C, Leu, B, Glu, Asn₂ et Pro peut être remplacé par Lys ou Orn.

3. Composé selon la revendication 1, dans lequel ledit polypeptide est la neurotensine ou un analogue de celle-ci.

4. Composé selon la revendication 1, dans lequel R représente Glu-Leu-Tyr-Glu-Asn-Lys-Pro-Arg-Arg-Pro-Tyr-Ile-Leu (SEQ ID N° 3), 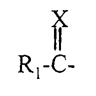 est une fluorescéine thiocarbamyle et R est lié par l'intermédiaire de la terminaison N de la position 1.

5. Composé de formule : 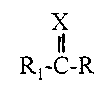 ou un sel d'acide pharmaceutiquement acceptable de celui-ci, dans lequel R est une séquence d'acide aminé comprenant au moins les résidus d'acide aminé 8-13 de la neurotensine, R₁ est un fluorophore photoémetteur; et X est un atome d'oxygène ou de soufre.

6. Composé selon l'une quelconque des revendications 1 à 5, dans lequel ledit fluorophore photoémetteur est choisi dans le groupe constitué par la fluorescéine, la rhodamine, le Bleu fluorescent et le rouge Texas.

7. Composé selon l'une quelconque des revendications 1 à 6, dans lequel ledit composé est homogène à au moins 99 %.

8. Méthode de marquage *in vitro* de récepteurs de la neurotensine sur une surface cellulaire, ladite méthode consistant à incuber des cellules dissociées, des préparations de cultures cellulaires ou de coupes tissulaires avec un composé selon l'une quelconque des revendications 1 à 7, et à visualiser lesdits composés par des méthodes de cytométrie en flux et/ou d'épifluorescence/microscopie confocale.

9. Méthode selon la revendication 8, dans laquelle ladite visualisation comprend en outre l'observation dudit composé et d'autres marqueurs fluorescents pour l'identification histochimique électrophysiologique, ou histochimique et électrophysiologique des cellules contenant le récepteur de la neurotensine.

10. Méthode permettant d'isoler les cellules du récepteur de la neurotensine, ladite méthode consistant : à incuber les cellules dissociées ou les préparations de cultures cellulaires avec un composé selon la revendication 1 ou 5, et à isoler les cellules liées audit composé par des méthodes de tri cellulaire à cytométrie en flux.
